# EUROPÄISCHE PATENTSCHRIFT

(11) **EP 3 634 896 B1**
(45) Veröffentlichungstag und Bekanntmachung des Hinweises auf die Patenterteilung: **11.08.2021**
(21) Anmeldenummer: 18729094.5
(22) Anmeldetag: 01.06.2018
(51) Int. Cl.: B65H 59/40, B65H 63/02, G01N 33/36, G01L 5/10, G01L 5/102

(54) **VERFAHREN UND VORRICHTUNG ZUR ÜBERWACHUNG EINER FADENSPANNUNG AN EINEM LAUFENDEN FADEN**
METHOD AND DEVICE FOR MONITORING A YARN TENSION OF A RUNNING YARN
PROCÉDÉ ET DISPOSITIF DE SURVEILLANCE D'UNE TENSION DU FIL SUR UN FIL EN COURS DE DÉPLACEMENT

(30) Priorität: 07.06.2017 DE 102017005450; 07.07.2017 DE 102017006431
(43) Veröffentlichungstag der Anmeldung: 15.04.2020
(73) Patentinhaber: Oerlikon Textile GmbH & Co. KG, 42897 Remscheid (DE)
(72) Erfinder: HUTHMACHER, Jörg, 45768 Marl (DE)
(74) Vertreter: Keenway Patentanwälte Neumann Heine Taruttis
(86) Internationale Anmeldenummer: PCT/EP2018/064413
(87) Internationale Veröffentlichungsnummer: WO 2018/224398

(56) Entgegenhaltungen:
- EP-A2- 2 107 027
- EP-A2- 2 644 551
- WO-A1-2015/052624
- DE-A1- 19 614 027
- US-A- 5 682 146

## Beschreibung

Die Erfindung betrifft ein Verfahren zur Überwachung einer Fadenspannung an einem laufenden Faden in einem Fadenbehandlungsprozess gemäß dem Oberbegriff des Anspruchs 1 sowie eine Vorrichtung zur Überwachung gemäß dem Oberbegriff des Anspruchs 7.

Ein gattungsgemäßes Verfahren und eine gattungsgemäße Vorrichtung zur Überwachung einer Fadenspannung an einem laufenden Faden in einem Fadenbehandlungsprozess ist beispielsweise aus der DE 196 14 027 A1 bekannt.

Bei der Herstellung und Behandlung von Fäden ist es üblich, Produkt und / oder Prozessparameter kontinuierlich zu überwachen, um so eine möglichst stabile Prozessführung und insbesondere möglichst eine stabile Produktqualität an dem Faden zu erhalten. Insbesondere bei der Herstellung von texturierten Fäden hat sich die Überwachung einer Fadenspannung an dem laufenden Faden bewährt, um Prozessstörungen und / oder Produktschwankungen zu erkennen. Bei dem bekannten Verfahren und der bekannten Vorrichtung zur Überwachung einer Fadenspannung an einem laufenden Faden wird hierzu die Fadenspannung an dem Faden fortlaufend gemessen. Die dabei erzeugten Messsignale der Fadenspannung werden mit einem Schwellwert einer zulässigen Fadenspannung verglichen. Hierbei können sogenannte Fehlergraphen erzeugt werden, die den Signalverlauf der Messsignale der Fadenspannung bei unzulässiger Toleranzabweichung aufzeigen. Somit lassen sich insbesondere Qualitätsschwankungen an dem Faden feststellen, die beispielsweise eine Qualitätseinstufung ermöglicht. Darüber hinaus ist bekannt, dass die Fehlergraphen in Abhängigkeit von der jeweiligen Störung im Prozess unterschiedliche Signalverläufe der Fadenspannung wiedergeben. So können erfahrene Operator den Signalverlauf der Fadenspannung eines Fehlergraphen nutzen, um eine mögliche Störquelle zu identifizieren.

Verfahren zur Überwachung der Fadenspannung sind aus der als nächstliegender Stand der Technik angesehenen US 5 682 146 A und der EP 2 107 027 A2 bekannt.

Es ist nun Aufgabe der Erfindung, das gattungsgemäße Verfahren und die gattungsgemäße Vorrichtung zur Überwachung einer Fadenspannung an einem laufenden Faden in einem Fadenbehandlungsprozess derart weiterzubilden, dass eine verbesserte Prozessführung zur Herstellung gleichmäßiger Fadenqualitäten möglich wird.

Ein weiteres Ziel der Erfindung liegt darin, ein gattungsgemäßes Verfahren und eine gattungsgemäße Vorrichtung zur Überwachung einer Fadenspannung an einem laufenden Faden in einem Fadenbehandlungsprozess zu schaffen, mit welchem es möglich ist, Prozessstörungen zu identifizieren sowie schnell und gezielt zu beseitigen.

Diese Aufgabe wird erfindungsgemäß durch ein Verfahren mit den Merkmalen nach Anspruch 1 und durch eine Vorrichtung mit den Merkmalen nach Anspruch 7 gelöst.

Vorteilhafte Weiterbildungen der Erfindung sind durch die Merkmale und Merkmalskombinationen der jeweiligen Unteransprüche definiert.

Die Erfindung basiert auf der Erkenntnis, dass die Fehlergraphen der Messsignalverläufe der Fadenspannung als Indiz einer Prozessstörung genutzt werden können. Um die Fehlergraphen als Grundlage für eine Diagnose zu verwenden, wird erfindungsgemäß der Fehlergraph mit einem maschinellen Lernprogramm analysiert. Danach wird der Fehlergraph einer bekannten Fehlergraphkategorie oder einer neuen Fehlergraphkategorie zugeordnet. Damit liegt eine eindeutige Klassifizierung des Fehlergraphens vor. Insbesondere die Zuordnung zu einer bekannten Fehlergraphkategorie ermöglicht eine frühzeitige Fehlerdiagnose, so dass zugrundeliegende Störursachen schnell erkannt werden. Alternativ ist es jedoch auch möglich, dass während des Prozesses sich bisher nicht bekannte oder bisher nicht berücksichtigte neue Prozessstörungen einstellen. In diesem Fall kann der Fehlergraph durch das maschinelle Lernprogramm einer neuen Fehlergraphkategorie zugeordnet werden.

Die ermittelten Fehlergraphen können zur Erweiterung eines Datenpools gespeichert.

Da die Fehlergraphen sich in ihrem Kurvenverlauf unterscheiden, besteht auch die Möglichkeit, die Fehlergraphkategorien durch ein Fehlermuster eines der Fehlergraphen oder einer Gruppe von Fehlergraphen der Fadenspannung zu spezifizieren. Dadurch lässt sich bei der Analyse der Fehlergraphen durch das maschinelle Lernprogramm eine Visualisierung der betreffenden Fehlergraphkategorie wesentlich verbessern.

Um nach der Zuordnung der Fehlergraphen zu einer der Fehlergraphkategorien eine schnelle und gezielte Prozessänderung durchzuführen, ist die Verfahrensvariante besonders vorteilhaft, bei welcher jedem der Fehlergraphkategorien eine bestimmte Prozessstörung und / oder ein bestimmter Bedienungsfehler und / oder ein bestimmter Störparameter und / oder ein bestimmter Produktfehler zugeordnet ist / sind. Damit kann nach Zuordnung der Fehlergraphen sofort die zugrundeliegende Ursache für die Fadenspannungsabweichung beseitigt werden. So lassen sich insbesondere bei der Herstellung der Fäden größere Ausschussmengen vermeiden.

Zur Automatisierung des jeweiligen Fadenbehandlungsprozesses ist die Verfahrensvariante vorteilhaft, bei welcher nach Zuordnung eines der Fehlergraphen zu einer der Fehlergraphkategorien ein die Fehlergraphkategorie betreffender Steuerungsbefehl zu einer Prozessänderung ausgelöst wird. Die Prozessänderung könnte beispielsweise ein direkter Eingriff in den Fadenbehandlungsprozess bewirken oder einen Eingriff durch einen Operator einleiten.

Da bei einem Fadenbehandlungsprozess eine Vielzahl von Fehlergraphen auftreten, hat sich die Verfahrensvariante besonders bewährt, bei welcher die Analyse der Fehlergraphen durch zumindest einen Maschinenlernalgorithmus des maschinellen Lernprogramms ausgeführt wird. So lässt sich eine künstliche Intelligenz dazu nutzen, um selbst bei einer großen Datenmenge strukturierte Analysen durchzuführen und bekannte und neue Fehlergraphkategorien schnell herauszufinden.

Hierbei ist es jedoch zunächst erforderlich, dass der Maschinenlernalgorithmus zum Lernen zunächst auf ermittelte Basisdaten zurückgreift. Hierzu werden Fehlergraphen, die einer der Fehlergraphkategorien zuvor zugewiesen wurden, dem Maschinenlernalgorithmus zum Lernen übergeben.

Nach einer Lernphase ist es möglich, dass der Maschinenlernalgorithmus durch Analyse eines vorgegeben Fehlergraphen selbsttätig diesen zumindest einer der vorhandenen Fehlergraphkategorien oder einer neuen Fehlergraphkategorie zuordnet. So können vorteilhaft auch neue Ursachen von Störungen im Prozess zukünftig erkannt und definiert werden.

Die erfindungsgemäße Vorrichtung lässt die Fadenspannungsauswertungseinheit unmittelbar mit einer Diagnoseeinheit zusammenwirken, so dass Fehleridentifizierung und Diagnose möglich wird. Die Diagnoseeinheit ermöglicht so zunächst eine Analyse eines Fehlergraphen mit einem maschinellen Lernprogramm und eine anschließende Zuordnung des Fehlergraphen zu einer bestehenden Fehlergraphkategorien oder einer neuen Fehlergraphkategorie.

Um die neu ermittelten Fehlergraphen für nachfolgende Analysen nutzen zu können, weist die Diagnoseeinheit eine Speichereinheit zur Speicherung der Fehlergraphen und einen programmierbaren Lernprozessor zur Ausführung des maschinellen Lernprogramms und anschließender Bestimmung der Fehlerkategorie auf.

Dabei könnte der Lernprozessor mit einer Eingabeeinheit gekoppelt sein, durch welche ein oder mehrerer ermittelte Fehlergraphen einlesbar sind. Damit können vorteilhaft auch bereits bekannte Fehlergraphkategorien der Diagnoseeinheit aufgebeben werden, um das maschinelle Lernprogramm zu verbessern.

Damit bei der Prozessführung ein Operator über den jeweiligen Prozesslauf informiert ist, ist des Weiteren vorgesehen, dass der Lernprozessor mit einer Ausgabeeinheit gekoppelt ist, durch welche eine Zuordnung der analysierten Fehlergraphen einer der Fehlergraphkategorien visualisierbar ist. Diese Ausgabeeinheit kann dabei vorteilhaft drahtlos mit dem Lernprozessor gekoppelt sein und jede Art von Gerät darstellen, mit welchem die Anzeige einer Fehlergraphkategorie möglich ist.

Um ein möglich autarkes System zur Fehlerdiagnose zu erhalten, ist des Weiteren vorgesehen, dass der Lernprozessor ein neuronales Netz zur Ausführung eines maschinellen Lernprogramms aufweist. So können auch komplexe Fadenbehandlungsprozesse mit entsprechend großer Datenvielfalt kontinuierlich und sicher überwacht werden.

Zu Überwachung mehrere Fadenbehandlungsprozesse ist die erfindungsgemäße Vorrichtung in der Weiterbildung vorteilhaft nutzbar, bei welchem der Lernprozessor räumlich getrennt zu der Eingabeeinheit und der Ausgabeeinheit angeordnet ist. Hierbei besteht die Möglichkeit, dass der Lernprozessor mit mehreren Eingabeeinheiten und/oder mehreren Ausgabeeinheiten in Kontakt steht. Die Verbindung erfolgt dann bevorzugt drahtlos, so dass der Lernprozessor auch in einem virtuellen Raum ausgebildet sein könnte.

Zur Automatisierung wird die erfindungsgemäße Vorrichtungsvariante vorteilhaft genutzt, bei welcher die Diagnoseeinheit mit einer Maschinensteuereinheit verbunden ist, durch welche ein Steuerbefehl zur Prozessänderung ausführbar ist. So können präzise Störursachen unmittelbar nach Zuordnung des jeweiligen Fehlergraphen beseitigt werden.

Das erfindungsgemäße Verfahren zur Überwachung einer Fadenspannung an einem laufenden in einem Fadenbehandlungsprozess wird nachfolgend an einem Ausführungsbeispiel der erfindungsgemäßen Vorrichtung unter Bezug auf die beigefügten Figuren näher erläutert.

Es stellen dar:
- Fig. 1: schematisch einen Fadenbehandlungsprozess mit einem Ausführungsbeispiel der erfindungsgemäßen Vorrichtung zur Überwachung einer Fadenspannung
- Fig. 2: schematisch das Ausführungsbeispiel der erfindungsgemäßen Vorrichtung zur Überwachung einer Fadenspannung aus Fig. 1
- Fig. 3.1 bis Fig. 3.5: mehrere unterschiedliche Fehlergraphen unterschiedlicher Fehlergraphkategorien

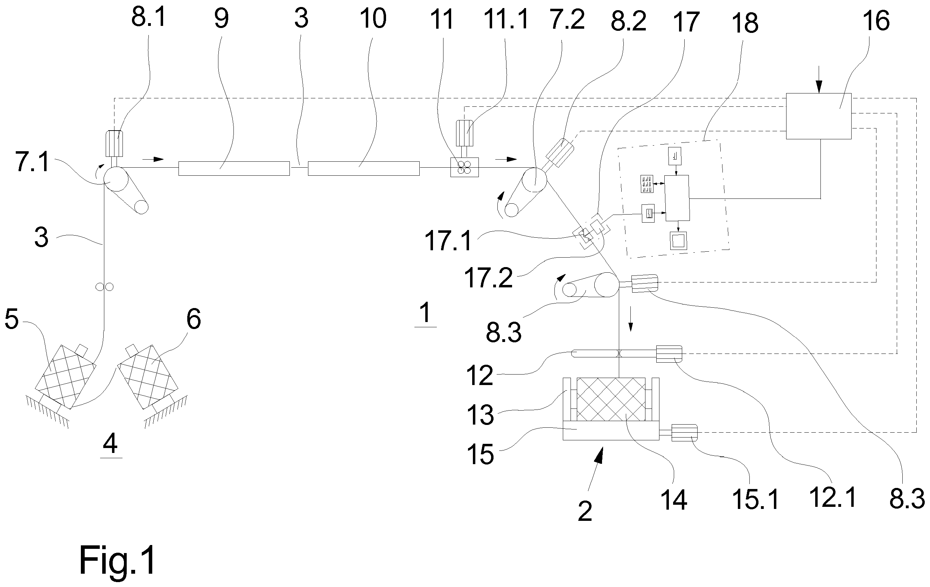

In der Fig. 1 ist schematisch ein Fadenbehandlungsprozess mit einem Ausführungsbeispiel der erfindungsgemäßen Vorrichtung gezeigt. Der Fadenbehandlungsprozess betrifft einen Texturierprozess, wie er in einer Vielzahl von Bearbeitungsstellen einer Texturiermaschine ausführbar ist. In Fig. 1 ist schematisch eine Bearbeitungsstelle 1 und eine Spulstelle 2 einer derartigen Texturiermaschine dargestellt. Die Bearbeitungsstelle 1 weist eine Vorlagestation 4 auf, in welcher eine Vorlagespule 5 und eine Reservespule 6 gehalten sind. Die Vorlagespule 5 liefert einen Faden 3, der zum Verstrecken und Texturieren in die Bearbeitungsstelle 1 überführt wird.

Der Abzug des Fadens von der Vorlagespule 5 erfolgt durch eine erste Galetteneinheit 7.1. Die Galetteneinheit 7.1 wird über einen Galettenantrieb 8.1 angetrieben. Im weiteren Verlauf ist der Galetteneinheit 7.1 eine Heizeinrichtung 9, eine Kühleinrichtung 10 und ein Texturieraggregat 11 nachgeordnet. Das Texturieraggregat 11 wird über einen Texturierantrieb 11.1 angetrieben. Das Texturieraggregat 11 ist vorzugsweise als Friktionsdrallgeber ausgebildet, um an dem Faden 3 einen Falschdrall zu erzeugen.

Zum Verstrecken des Fadens ist dem Texturieraggregat 11 eine zweite Galetteneinheit 7.2 nachgeordnet, die durch den Galettenantrieb 8.2 betrieben wird. Die Galetteneinheit 7.2 ist im Aufbau identisch zu der ersten Galetteneinheit 7.1, wobei die zweite Galetteneinheit 7.2 mit einer höheren Umfangsgeschwindigkeit zum Verstrecken des Fadens betrieben wird. Innerhalb der Bearbeitungsstelle 1 wird der Faden 3 somit texturiert und gleichzeitig verstreckt. Nach der Behandlung des Fadens wird dieser durch eine dritte Galetteneinheit 7.3 zu einer Spulstelle 2 geführt. Die Galetteneinheit 7.3 wird durch den Galettenantrieb 8.3 angetrieben.

Die Spulstelle 2 weist einen Spulenhalter 13 auf, welcher eine Spule 14 trägt. Der Spulenhalter 13 ist schwenkbar ausgebildet und lässt sich zum Auswechseln der Spule 14 manuell oder automatisiert bedienen. Dem Spulhalter 13 ist eine Treibwalze 15 zugeordnet, die durch einen Walzenantrieb 15.1 angetrieben wird. Zum Verlegen des Fadens am Umfang der Spule 15 ist der Spulstelle 2 eine Changiereinheit 12 zugeordnet, die einen antreibbaren Changierfadenführer aufweist. Der Changierfadenführer wird hierzu über den Changierantrieb 12.1 angetrieben.

Der Changierantrieb 12.1 und der Walzenantrieb 15.1 der Spulstelle 2 sind als Einzelantriebe ausgebildet und mit einer Maschinensteuereinheit 16 verbunden. Ebenso sind die Galettenantriebe 8.1, 8.2 und 8.3 sowie der Texturierantrieb 11.1 der Bearbeitungsstelle 1 als Einzelantriebe ausgeführt und mit der Maschinensteuereinheit 16 gekoppelt.

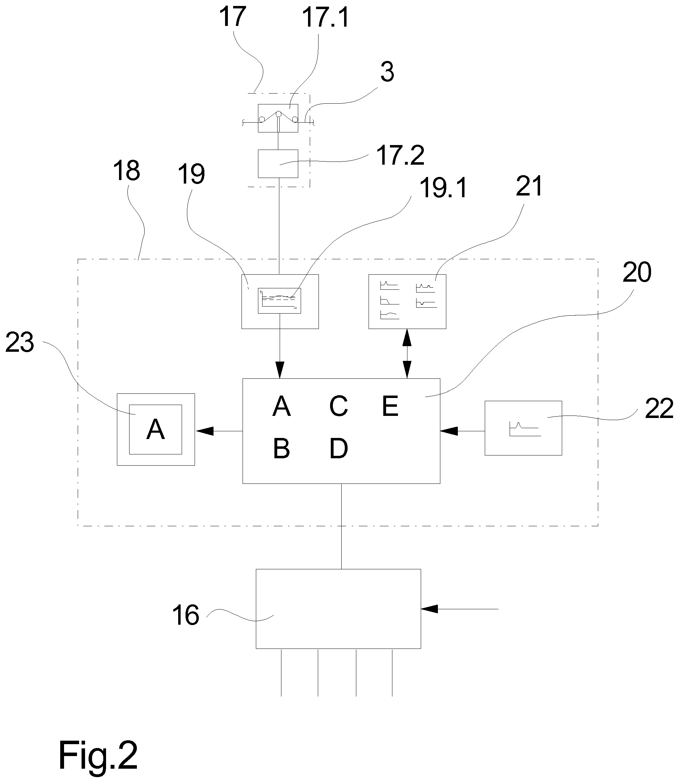

Zur Prozessüberwachung wird an dem laufenden Faden 3 in einem Fadenabschnitt zwischen den Galetteneinheiten 7.2 und 7.3 kontinuierlich eine Fadenspannung gemessen und überwacht. Hierzu ist eine Fadenspannungsmesseinrichtung 17 vorgesehen, die einen Fadenspannungssensor 17.1 und einen Messsignalaufnehmer 17.2 aufweist. Der Fadenspannungsmesseinrichtung 17 ist mit einer Diagnoseinheit 18 verbunden. Zur weiteren Erläuterung der Diagnoseeinheit 18 wird zusätzlich zu der Fig. 2 Bezug genommen.

In der Fig. 2 ist schematisch die erfindungsgemäße Vorrichtung zur Überwachung einer Fadenspannung an einem laufenden Faden dargestellt. Die Diagnoseeinheit 18 umfasst hierbei die Fadenspannungsauswertungseinheit 19, die direkt mit der Fadenspannungsmesseinrichtung 17 verbunden ist. So werden die Messsignale des Messsignalaufnehmers 17.2 der Fadenspannungsauswertungseinheit 19 zugeführt. Innerhalb der Fadenspannungsauswertungseinheit 19 werden die Messsignale aufbereitet und mit zumindest einem Grenzwert verglichen. Üblicherweise wird das Messsignal an der Fadenspannung mit einem oberen Grenzwert und einem unteren Grenzwert verglichen. Sobald eine unzulässige Toleranzabweichung der Fadenspannung erkannt wird, wird ein kurzzeitiger Messsignalverlauf der Fadenspannung aufgezeichnet und als ein Fehlergraph erzeugt. Hierzu weist die Fadenspannungsauswertungseinheit 19 einen Fehlergrapherzeuger 19.1 auf. Der Fehlergraph wird an einem Lernprozessor 20 übergeben. Der Lernprozessor 20 ist programmierbar ausgeführt und weist vorzugsweise ein neuronales Netz auf, um ein maschinelles Lernprogramm auszuführen. Das maschinelle Lernprogram umfasst zumindest einen Maschinenlernalgorithmus, um mit künstlicher Intelligenz umfangreiche Analysen durchführen zu können.

Dem Lernprozessor 20 ist eine Speichereinheit 21 zugeordnet, in welcher ein Datenpool von Fehlergraphen gespeichert ist. Desweiteren sind dem Lernprozessor 20 eine Eingabeeinheit 22 und eine Ausgabeeinheit 23 zugeordnet.

Die Verbindungen zwischen dem Lernprozessor 20 und der Fadenspannungsmesseinrichtung 17, der Speichereinheit 21, der Eingabeeinheit 22 und der Ausgabeeinheit 23 kann durch jeweils eine drahtgebundene oder drahtlose Verbindung erfolgen. Insbesondere bei einer drahtlosen Verbindung besteht die Möglichkeit, dass die einzelnen Einheiten nicht unbedingt am selben Ort gehalten werden müssen. So ist der Lernprozessor 20 bevorzugt drahtlos in der Diagnoseeinheit 18 integriert. Dabei besteht auch die Möglichkeit den Lernprozessor 20 unabhängig von der Eingabeeinheit 22 und der Ausgabeeinheit in einem virtuellen Raum anzuordnen.

In dem Lernprozessor 20 wird der von dem Fehlergrapherzeuger 19.1 erzeugter Fehlergraph mit dem maschinellen Lernprogramm analysiert. Hierzu weist das maschinelle Lernprogramm zumindest einen Maschinenlernalgorithmus auf, der mit Hilfe eines neuronalen Netzes eine strukturierte Analyse des Fehlergraphen zur Identifizierung einer Fehlergraphkategorie ausführt. Eine Fehlergraphkategorie kann hierbei durch ein einziges Fehlermuster eines der Fehlergraphen oder einer Gruppe von Fehlergraphen spezifiziert sein.

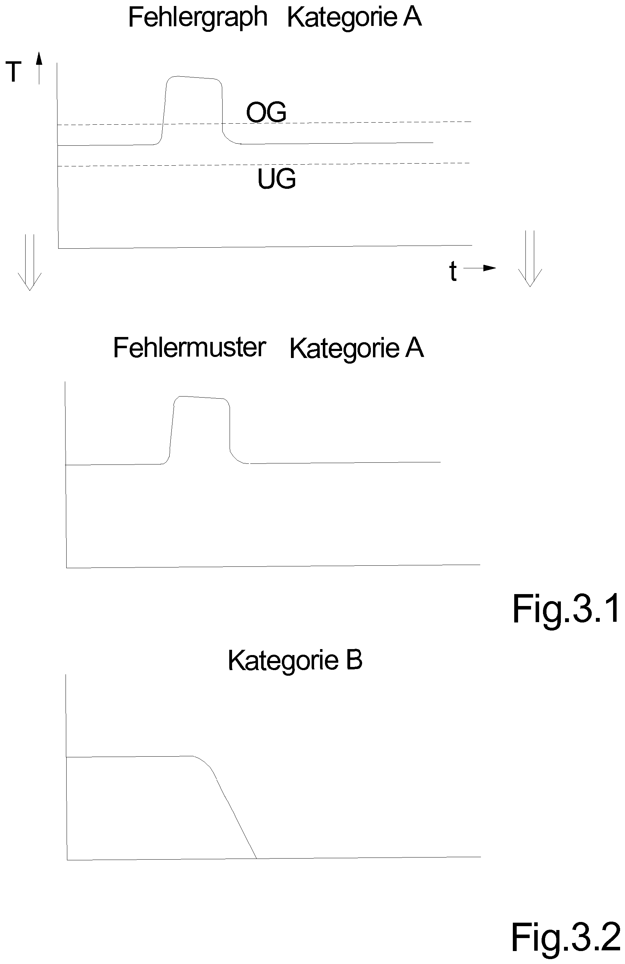

In den Fig. 3.1 bis 3.5 sind einige Ausführungsbeispiele von Fehlermustern typischer Fehlergraphen und Fehlergraphkategorien dargestellt. Jeder der in den Fig. 3.1 bis 3.5 dargestellten Fehlermuster bildet einen Fehlergraph nach, der einer bestimmten Fehlergraphkategorie zugeordnet ist.

In der Fig. 3.1 ist hierzu beispielhaft in der oberen Bildhälfte der Verlauf des Messsignals der Fadenspannung als Fehlergraph dargestellt. Das Messsignal der Fadenspannung wird in diesem Fall mit einem oberen Grenzwert und einem unteren Grenzwert verglichen. In dem Fehlergraph ist der obere Grenzwert mit dem Kennbuchstaben OG und der untere Grenzwert mit dem Buchstaben UG gekennzeichnet. Hierzu ist auf der Ordinate die Fadenspannung T und auf der Abszisse die Zeit t aufgetragen. Bei dem in Fig. 3.1 dargestellten Signalverlauf der Fadenspannung ist eine kurzzeitige Überschreitung des oberen Grenzwertes zu erkennen. Diese sehr plötzliche Überschreitung hat ihre Ursache in einem sogenannten Knotenüberlauf. So geht aus dem Ausführungsbeispiel nach Fig. 1 hervor, dass ein Ende der Vorlagespule mit einem Anfang der Reservespule verknotet ist, um einen kontinuierlichen Prozess zu ermöglichen. Dieser Verbindungsknoten im Fadenlauf führt am Fadenspannungssensor 17.1 zu einer kurzzeitigen Fadenspannungserhöhung. Insbesondere für den Weiterverarbeitungsprozess ist die Information von großer Bedeutung, ob eine Endspule einen Knoten und somit das Vorlagematerial von zwei Vorlagespulen enthält.

In der Fig. 3.1 ist der in der oberen Bildhälfte dargestellte Fehlergraph als ein Fehlermuster in der unteren Bildhälfte dargestellt. Das in Fig. 3.1 in der unteren Bildhälfte dargestellte Fehlermuster könnte beispielsweise eine Fehlergraphkategorie A darstellen. In der späteren Prozessführung lässt sich somit durch Analyse der Fehlergraphen die Fehlergraphkategorie A ermitteln und möglicherweise mit dem Fehlermuster einem Operator gegenüber visualisieren.

In den Fig. 3.2 bis 3.5 sind weitere Fehlermuster dargestellt, die jeweils eine bestimmte Fehlergraphkategorie bestimmen.

Das in Fig. 3.2 dargestellte Fehlermuster bestimmt die Fehlergraphkategorie B. Die Fehlerkategorie B könnte ihre Störursache in einem Fadenbruch oder einem Bedienfehler, da die Fadenspannung an dem Fadenspannungssensor plötzlich komplett einbricht.

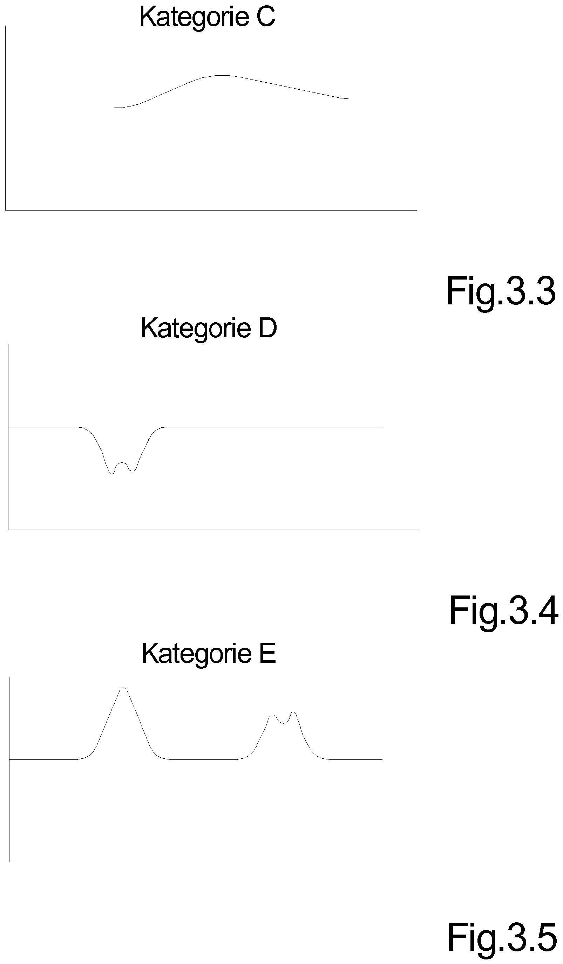

Das in Fig. 3.3 dargestellte Fehlermuster bestimmt die Fehlergraphkategorie C. Die Fehlergraphkategorie C könnte beispielsweise eine Verschleißsituation an den Prozessaggregaten beispielsweise einer vom Faden kontaktierten Kühlschiene darstellen.

Bei dem in Fig. 3.4 dargestellten Fehlermustern liegt die Toleranzabweichung des Messsignals des Fehlergraphen am unteren Grenzwert. Dies deutet auf einen kurzzeitigen Fadenspannungsverlust hin. Das zugehörige Fehlermuster definiert in diesem Fall die Fehlergraphkategorie D. Hier könnte eine Prozessstörung beim Spulenwechsel in der Spulstelle 2 vorliegen.

In dem in Fig. 3.5 dargestellten Fehlermuster ist eine wiederkehrende Überschreitung des Messsignals der Fadenspannung gegenüber dem oberen Grenzwert gemessen. Das zugehörige Fehlermuster bildet in diesem Fall die Fehlergraphkategorie E. Die Fehlergraphkategorie E könnte beispielsweise in einer ungleichmäßigen Texturierung des Fadens liegen.

Insoweit ist jedem der Fehlergraphkategorien A bis E eine bestimmte Prozessstörung oder ein bestimmter Bedienungsfehler oder ein bestimmter Produktfehler zugeordnet. Die in den Fig. 3.1 bis 3.5 dargestellten Fehlergraphkategorien sind beispielhaft. In einem Fadenbehandlungsprozess, wie in Fig. 1 dargestellt, kann eine Vielzahl von Fehlergraphkategorien auftreten, denen eine eindeutige Ursache im Prozess zugrunde liegt.

Bei der in Fig. 2 dargestellten Diagnoseeinheit 18 wird innerhalb des Lernprozessors 20 der von der Fadenspannungsauswertungseinheit 19 bereitgestellte Fehlergraph mit dem maschinellen Lernprogramm analysiert. Hierbei wird der Fehlergraph mit Hilfe von zumindest einem vorzugsweise mehreren Maschinenlernalgorithmen analysiert. Am Ende der Analyse erfolgt eine Klassifizierung in eine der bereits gelernten Fehlergraphkategorien. Falls es sich hierbei um eine noch nicht bekannte Fehlergraphkategorie handelt, wird eine neue Fehlerkategorie geschaffen und innerhalb des maschinellen Lernprogramms des Lernprozessors aufgenommen.

Zur Ausführung der Operationen weist der Lernprozessor ein neuronales Netz auf, um den Maschinenlernalgorithmus ausführen zu können. Die Diagnoseeinheit ist somit völlig autark, um die Fehlergraphen zu analysieren und damit die Fadenspannung zu überwachen und entsprechende Maßnahmen zur Behebung von Störungen einzuleiten.

Bei dem Ausführungsbeispiel nach Fig. 2 hat die aktuelle Analyse der Fehlergraphen eine Zuordnung zur Fehlergraphkategorie A ergeben. Diese Fehlergraphkategorie wird an der Ausgabeeinheit 23 beispielsweise durch das Fehlermuster visualisiert, so dass ein Operator informiert ist und ggf. selbst Handlungen durchführen kann. Alternativ könnte jedoch durch den Lernprozessor 20 unmittelbar ein Signal an die Maschinensteuereinheit 16 gegeben werden. Daher ist der Lernprozessor 20 auch mit der Maschinensteuereinheit 16 verbunden. So könnte beispielsweise bei der Fehlergraphkategorie A ein vorzeitiger Spulenwechsel eingeleitet werden, um in der gewickelten Spule keine Knotenverbindung zu enthalten.

Im späteren Verlauf des Prozesses ist die Diagnoseeinheit 18 in der Lage auch neue bisher nicht bekannte Fehlerkategorien zu generieren. Die Diagnoseeinheit 18 stellt somit ein Selbstlernsystem dar, durch welches eine automatisierte Klassifizierung in verschiedenen Kategorien erfolgt. Jede der Fehlergraphkategorien könnte dabei eine bestimmte Prozessstörung darstellen, so dass den erfassten Fehlergraphen automatisiert über die Fehlergraphkategorie ein Störparameter zugeordnet werden könnte. Durch den Zusammenhang zwischen Fehlergraphkategorie und Störparameter könnten somit schnelle und eindeutige Fehlerdiagnosen in dem Texturierprozess ausgeführt und durch entsprechende Reaktionen automatisiert beseitigt werden.

## Patentansprüche

1. Verfahren zur Überwachung einer Fadenspannung an einem laufenden Faden in einem Fadenbehandlungsprozess, bei welchem die Fadenspannung des Fadens fortlaufend gemessen wird, bei welchem Messsignale der Fadenspannung mit zumindest einem Grenzwert einer zulässigen Fadenspannung verglichen werden und bei welchem bei einer unzulässigen Toleranzabweichung der Messsignale ein kurzzeitiger Signalverlauf der Fadenspannung als ein Fehlergraph erfasst wird, **dadurch gekennzeichnet, dass** der Fehlergraph der Fadenspannung mit einem maschinellen Lernprogramm analysiert wird und dass der Fehlergraph einer bekannten Fehlergraphkategorie oder einer neuen Fehlergraphkategorie zugeordnet wird.

2. Verfahren nach Anspruch 1, **dadurch gekennzeichnet, dass** die Fehlergraphkategorien jeweils durch ein Fehlermuster eines der Fehlergraphen und/oder einer Gruppe von Fehlergraphen spezifiziert sind.

3. Verfahren nach einem der Anspruch 1 oder 2, **dadurch gekennzeichnet, dass** jedem der Fehlergraphkategorien eine bestimmte Prozessstörung und/oder ein bestimmter Bedienungsfehler und/oder ein bestimmter Störparameter und/oder ein bestimmter Produktfehler zugeordnet ist/sind.

4. Verfahren nach Anspruch 3, **dadurch gekennzeichnet, dass** nach Zuordnung eines der Fehlergraphen zu einer der Fehlergraphkategorien ein die Fehlergraphkategorie betreffender Steuerungsbefehl zu einer Prozessänderung ausgelöst wird.

5. Verfahren nach Anspruch einem der Ansprüche 1 bis 4, **dadurch gekennzeichnet, dass** die Analyse der Fehlergraphen durch zumindest einen Maschinenlernalgorithmus des maschinellen Lernprogramms ausgeführt wird.

6. Verfahren nach Anspruch 5, **dadurch gekennzeichnet, dass** zumindest einer der Fehlergraphkategorien allein durch den Maschinenlernalgorithmus aus analysierten Fehlergraphen bestimmt ist.

7. Vorrichtung zur Überwachung einer Fadenspannung an einem laufenden Faden in einem Fadenbehandlungsprozess, insbesondere zur Durchführung des Verfahrens nach einem der Ansprüche 1 bis 6, aufweisend eine Fadenspannungsmesseinrichtung (17) mit einem Fadenspannungssensor (17.1) und mit einem Messsignalaufnehmer (17.2) und eine Fadenspannungsauswertungseinheit (19) mit einem Fehlergrapherzeuger (19.1), **dadurch gekennzeichnet, dass** die Fadenspannungsauswertungseinheit (19) mit einer Diagnoseeinheit (18) derart zusammenwirkt, dass ein Fehlergraph mit einem maschinellen Lernprogramm analysierbar ist und dass dem Fehlergraphen eine bekannte Fehlergraphkategorien oder eine neue Fehlergraphkategorie zugeordnet wird.

8. Vorrichtung nach Anspruch 7, **dadurch gekennzeichnet, dass** die Diagnoseeinheit (18) eine Speichereinheit (21) und einen programmierbaren Lernprozessor (20) zur Ausführung des maschinellen Lernprogramms aufweist.

9. Vorrichtung nach Anspruch 8, **dadurch gekennzeichnet, dass** der Lernprozessor (20) mit einer Eingabeeinheit (22) gekoppelt ist, durch welche ein oder mehrere ermittelte Fehlergraphen einlesbar sind.

10. Vorrichtung nach Anspruch 8 oder 9, **dadurch gekennzeichnet, dass** der Lernprozessor (20) mit einer Ausgabeeinheit (23) gekoppelt ist, durch welche eine Zuordnung der analysierten Fehlergraphen einer der Fehlergraphkategorien visualisierbar ist.

11. Vorrichtung nach einem der Ansprüche 8 bis 10, **dadurch gekennzeichnet, dass** der Lernprozessor (20) ein neuronales Netz zur Ausführung des maschinellen Lernprogramms aufweist.

12. Vorrichtung nach einem der Ansprüche 7 bis 12, **dadurch gekennzeichnet, dass** die Diagnoseeinheit (18) mit einer Maschinensteuereinheit (16) verbunden ist, durch welche ein Steuerbefehl zur Prozessänderung ausführbar ist.

## Claims

1. A method for monitoring a yarn tension of a running yarn in a yarn treatment process, in which the yarn tension of the yarn is progressively measured, in which measurement signals of the yarn tension are compared to at least one limiting value of a permissible yarn tension and in which in the event of an impermissible tolerance deviation of the measurement signals, a short-term signal profile of the yarn tension is acquired as a fault graph, **characterized in that** the fault graph of the yarn tension is analyzed using a machine learning program, and **in that** the fault graph is assigned to a known fault graph category or a new fault graph category.

2. The method as claimed in claim 1, **characterized in that** the fault graph categories are each specified by a fault pattern of one of the fault graphs and/or a group of fault graphs.

3. The method as claimed in one of claims 1 or 2, **characterized in that** a specific process disturbance and/or a specific operating fault and/or a specific disturbance parameter and/or a specific product fault is/are assigned to each of the fault graph categories.

4. The method as claimed in claim 3, **characterized in that** after assignment of one of the fault graphs to one of the fault graph categories, a control command relating to the fault graph category is triggered for a process change.

5. The method as claimed in claim any one of claims 1 to 4, **characterized in that** the analysis of the fault graphs is executed by at least one machine learning algorithm of the machine learning program.

6. The method as claimed in claim 5, **characterized in that** at least one of the fault graph categories is defined solely by the machine learning algorithm from analyzed fault graphs.

7. A device for monitoring a yarn tension of a running yarn in a yarn treatment process, in particular for carrying out the method as claimed in any one of claims 1 to 6, comprising a yarn tension measuring unit (17) having a yarn tension sensor (17.1) and having a measurement signal pickup (17.2) and a yarn tension analysis unit (19) having a fault graph generator (19.1), **characterized in that** the yarn tension analysis unit (19) interacts with a diagnostic unit (18) of the device in such a way that a fault graph is analyzable using a machine learning program, and **in that** a known fault graph category or a new fault graph category is assigned to the fault graph.

8. The device as claimed in claim 7, **characterized in that** the diagnostic unit (18) comprises a storage unit (21) and a programmable learning processor (20) for executing the machine learning program.

9. The device as claimed in claim 8, **characterized in that** the learning processor (20) is coupled to an input unit (22), by which one or more ascertained fault graphs can be input.

10. The device as claimed in claim 8 or 9, **characterized in that** the learning processor (20) is coupled to an output unit (23), by which an assignment of the analyzed fault graphs to one of the fault graph categories can be visualized.

11. The device as claimed in any one of claims 8 to 10, **characterized in that** the learning processor (20) comprises a neural network for executing the machine learning program.

12. The device as claimed in any one of claims 7 to 11, **characterized in that** the diagnostic unit (18) is connected to a machine control unit (16), by which a control command for the process change is executable.

## Revendications

1. Procédé permettant de surveiller une tension de fil sur un fil en mouvement dans un processus de traitement de fil, dans lequel la tension de fil du fil est mesurée en continu, des signaux de mesure de la tension de fil sont comparés avec au moins une valeur limite d'une tension de fil admissible, et en cas d'écart de tolérance inadmissible des signaux de mesure, un trajet de signal instantané de la tension de fil est détecté en tant que graphique d'erreur, **caractérisé en ce que** le graphique d'erreur de la tension de fil est analysé par un programme d'apprentissage machine, et **en ce que** le graphique d'erreur est associé à une catégorie de graphique d'erreur connue ou à une nouvelle catégorie de graphique d'erreur.

2. Procédé selon la revendication 1, **caractérisé en ce que** les catégories de graphiques d'erreur sont spécifiées respectivement par un schéma d'erreur d'un des graphiques d'erreur et/ou d'un groupe de graphiques d'erreur.

3. Procédé selon l'une quelconque des revendications 1 ou 2, **caractérisé en ce qu'**une certaine anomalie de processus et/ou une certaine erreur de manipulation et/ou un certain paramètre perturbateur et/ou un certain défaut de produit sont associés à chacune des catégories de graphiques d'erreur.

4. Procédé selon la revendication 3, **caractérisé en ce qu'**après l'association d'un des graphiques d'erreur à l'une des catégories de graphiques d'erreur, une instruction de commande concernant la catégorie de graphique d'erreur est déclenchée pour une modification de processus.

5. Procédé selon l'une quelconque des revendications 1 à 4, **caractérisé en ce que** l'analyse des graphiques d'erreur est réalisée par au moins un algorithme d'apprentissage machine du programme d'apprentissage machine.

6. Procédé selon la revendication 5, **caractérisé en ce qu'**au moins l'une des catégories de graphique d'erreur est déterminée uniquement par l'algorithme d'apprentissage machine parmi des graphiques d'erreur analysés.

7. Dispositif permettant de surveiller une tension de fil sur un fil en mouvement dans un processus de traitement de fil, en particulier pour effectuer le procédé selon l'une quelconque des revendications 1 à 6, présentant un équipement de mesure de tension de fil (17) doté d'un capteur de tension de fil (17.1) et d'un capteur de signal de mesure (17.2) et une unité d'évaluation de tension de fil (19) dotée d'un générateur de graphique d'erreur (19.1), **caractérisé en ce que** l'unité d'évaluation de tension de fil (19) coopère avec une unité de diagnostic (18) de telle sorte qu'un graphique d'erreur peut être analysé par un programme d'apprentissage machine et qu'une catégorie de graphique d'erreur connue ou une nouvelle catégorie de graphique d'erreur est associée au graphique d'erreur.

8. Dispositif selon la revendication 7, **caractérisé en ce que** l'unité de diagnostic (18) présente une unité de stockage (21) et un processeur d'apprentissage programmable (20) pour effectuer le programme d'apprentissage machine.

9. Dispositif selon la revendication 8, **caractérisé en ce que** le processeur d'apprentissage (20) est couplé à une unité d'entrée (22) par laquelle un ou plusieurs graphiques d'erreur déterminés peuvent être lus.

10. Dispositif selon la revendication 8 ou 9, **caractérisé en ce que** le processeur d'apprentissage (20) est couplé à une unité de sortie (23) par laquelle une association des graphiques d'erreur analysés d'une des catégories de graphiques d'erreur peut être visualisée.

11. Dispositif selon l'une quelconque des revendications 8 à 10, **caractérisé en ce que** le processeur d'apprentissage (20) présente un réseau neuronal pour effectuer le programme d'apprentissage machine.

12. Dispositif selon l'une quelconque des revendications 7 à 12, **caractérisé en ce que** l'unité de diagnostic (18) est reliée à une unité de commande machine (16) par laquelle une instruction de commande pour une modification de processus peut être exécutée.
